# FASCICULE DE BREVET EUROPEEN

(11) **EP 3 890 665 B1**
(45) Date de publication et mention de la délivrance du brevet: **15.05.2024**
(21) Numéro de dépôt: 19813531.1
(22) Date de dépôt: 04.12.2019
(51) Int. Cl.: A61F 5/02

(54) **CEINTURE ORTHOPÉDIQUE AVEC ÉLÉMENTS DE PRÉHENSION**
ORTHOPÄDISCHES BAND MIT GREIFELEMENTEN
ORTHOPAEDIC BELT WITH GRIPPING ELEMENTS

(30) Priorité: 05.12.2018 FR 1872350
(43) Date de publication de la demande: 13.10.2021
(73) Titulaire: Thuasne, 92300 Levallois Perret (FR)
(72) Inventeur: PANNETIER, Romain, 69100 Villeurbanne (FR); BERNOUX, Grégoire, Boris, 42000 SAINT-ETIENNE (FR); VOLDOIRE, Marie-Thérèse, 42100 SAINT-ETIENNE (FR)
(74) Mandataire: Lavoix
(86) Numéro de dépôt international: PCT/EP2019/083668
(87) Numéro de publication internationale: WO 2020/115127

(56) Documents cités:
- FR-A1- 2 952 807
- FR-A1- 2 987 739
- US-A1- 2009 118 655
- US-A1- 2010 217 167
- US-A1- 2014 364 786

## Description

La présente invention concerne une ceinture orthopédique pour un utilisateur, telle que définie par la revendication indépendante 1, ladite ceinture comprenant un premier panneau latéral et un deuxième panneau latéral, chacun du premier panneau latéral et du deuxième panneau latéral présentant une extrémité dorsale et une extrémité distale, les premier et deuxième panneaux latéraux étant destinés à entourer ensemble au moins une partie du corps de l'utilisateur, la ceinture définissant une ligne centrale adaptée pour être placée contre la colonne vertébrale de l'utilisateur, la ligne centrale définissant une direction principale, les extrémités dorsales des premier et deuxième panneaux latéraux étant reliées l'une à l'autre, les extrémités distales des premier et deuxième panneaux latéraux étant adaptées pour être fixées l'une à l'autre de manière réversible.

Une ceinture orthopédique permet notamment de soutenir ou assister la colonne vertébrale selon les besoins de l'utilisateur.

La géométrie de la ceinture orthopédique est alors adaptée auxdits besoins pour fournir l'effort ou le soutien souhaité.

Cependant, la forme de la ceinture est susceptible de rendre difficile le placement de la ceinture autour de l'utilisateur sans une aide extérieure. En particulier, pour une ceinture présentant une asymétrie, le placement de l'arrière de la ceinture par rapport à la colonne vertébrale d'un utilisateur est susceptible d'être mal réalisé.

Ainsi, une telle ceinture orthopédique est susceptible d'être mal portée et ainsi de ne pas avoir l'utilité prévue, voire d'être néfaste pour l'utilisateur.

Un but de l'invention est donc de proposer une ceinture orthopédique adaptée pour apporter un soutien à un utilisateur et facile à mettre en place par un utilisateur. Documents FR 2 987 739 A1 et US 2010/217167 A1 décrivent une ceinture orthopédique.

A cet effet, l'invention a pour objet une ceinture du type précitée, dans laquelle les premier et deuxième panneaux latéraux sont asymétriques par rapport à la ligne centrale, de sorte que les extrémités distales sont configurées pour être placées asymétriquement autour de l'utilisateur, le premier panneau latéral et le second panneau latéral étant chacun pourvus d'un élément de préhension pour l'utilisateur pour positionner la ceinture autour de l'utilisateur, les éléments de préhension étant disposés symétriquement sur la ceinture par rapport à la ligne centrale.

La présence des éléments de préhension placés symétriquement permet de symétriser la prise, bien que la ceinture orthopédique soit de forme générale asymétrique. Cela permet alors à l'utilisateur de se représenter correctement le placement de la ceinture et ainsi de la positionner correctement.

La ceinture orthopédique peut en outre présenter une ou plusieurs des caractéristiques ci-dessous, considérée(s) individuellement ou selon toutes les combinaisons techniquement possibles :
- le rapport entre la distance moyenne entre la ligne centrale et chaque extrémité distale et la distance entre la ligne centrale et chaque élément de préhension est compris entre 1,5 et 1,75,
- chaque élément de préhension comprend une bande textile s'étendant entre une première extrémité et une deuxième extrémité, la bande textile étant liée au premier ou deuxième panneau latéral respectif aux première et deuxième extrémités,
- la bande textile est libre du premier ou deuxième panneau latéral respectif en dehors des première et deuxième extrémités de la bande textile,
- les première et deuxième extrémités de la bande textile sont sensiblement alignées selon la direction principale,
- les première et deuxième extrémités de la bande textile s'étendent selon des axes respectifs non parallèles l'un à l'autre, la dimension de la bande textile selon la direction principale diminuant en s'approchant de l'extrémité distale du premier ou deuxième panneau latéral respectif,
- la ceinture comprend un mécanisme de serrage comprenant :
   - une rangée supérieure de guides de câble sur chacune des extrémités dorsales des premier et deuxième panneaux latéraux,
   - une rangée inférieure de guides de câble sur chacune des extrémités dorsales des premier et deuxième panneaux latéraux,
   - un câble enfilé à travers les guides de câble des rangées supérieure et inférieure du premier panneau latéral et du deuxième panneau latéral, le câble étant enfilé alternativement à travers les guides de câble du premier panneau latéral et du deuxième panneau latéral,
   - un premier et un deuxième éléments de poignée,
      le câble étant relié aux premier et deuxième éléments de poignée, de sorte que les extrémités dorsales des premier et deuxième panneaux latéraux sont rapprochées par serrage du câble autour des guides de câble des rangées supérieure et inférieure lorsqu'au moins un du premier et du deuxième éléments de poignée est tiré,
      les extrémités du câble étant fixées de manière au moins sensiblement fixe au premier élément de poignée, le câble étant enfilé de manière coulissante à travers le deuxième élément de poignée,
- la rangée supérieure de guides de câble du premier panneau latéral est espacée de la rangée inférieure de guides de câble du premier panneau latéral par un espace et la rangée supérieure de guides de câble du deuxième panneau latéral est espacée de la rangée inférieure de guides de câble du deuxième panneau latéral par un espace, et/ou
- le câble est unique.

L'invention concerne en outre une méthode de mise en place d'une ceinture orthopédique telle que définie précédemment, comprenant les étapes suivantes :
- saisie par un utilisateur de la ceinture par les éléments de préhension,
- placement de la ceinture autour d'une partie du corps de l'utilisateur par placement des éléments de préhension symétriquement de part et d'autre de ladite partie du corps, et
- attache de la ceinture autour du corps de l'utilisateur par fixation des extrémités distales l'une à l'autre.

D'autres caractéristiques et avantages de l'invention apparaîtront à la lecture de la description qui suit de modes de réalisation de l'invention, donnée à titre d'exemple uniquement et en référence aux dessins dans lesquels :
[Fig. 1] la figure 1 est une vue schématique d'une ceinture selon un mode de réalisation de l'invention,

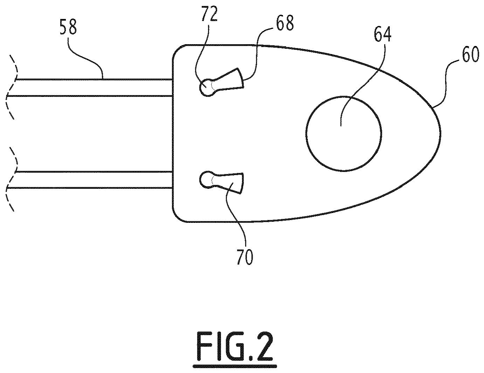

[Fig. 2] la figure 2 est une vue schématique d'un premier élément de poignée de la ceinture de la figure 1, et

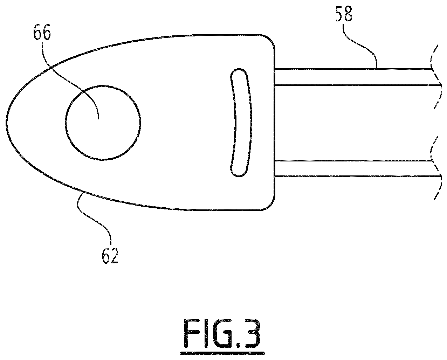

[Fig. 3] la figure 3 est une vue schématique d'un deuxième élément de poignée de la ceinture de la figure 1.

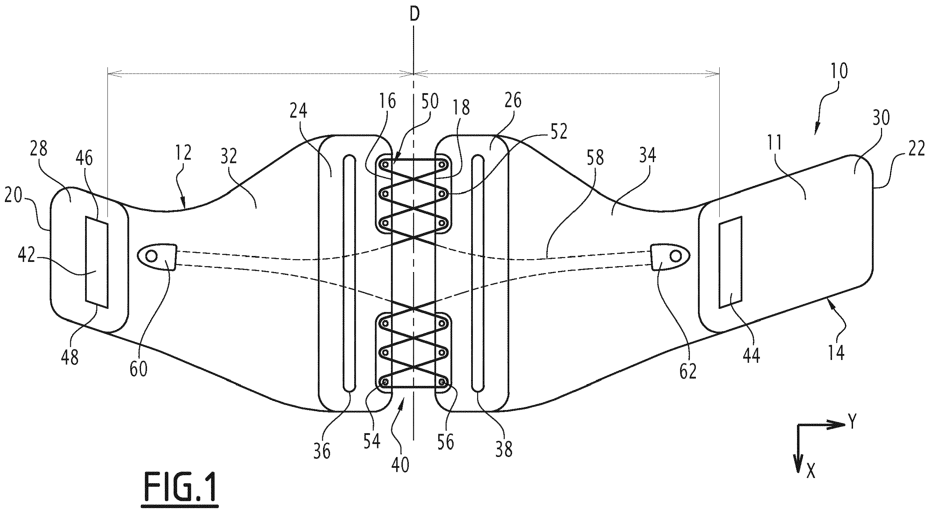

Une ceinture orthopédique 10 pour un utilisateur selon un mode de réalisation de l'invention est représentée sur la figure 1.

La ceinture est prévue pour entourer le tronc d'un utilisateur lorsque la ceinture 10 est portée par cet utilisateur.

La ceinture 10 est prévue pour appliquer une pression sur un utilisateur lorsqu'il entoure le tronc dudit utilisateur, de manière à soutenir le tronc de l'utilisateur, notamment pour prévenir ou soulager un mal de dos.

La ceinture 10 définit une ligne centrale D adaptée pour être placée contre la colonne vertébrale de l'utilisateur.

La ligne centrale D s'étend selon une direction principale X.

La ceinture 10 présente une face interne non visible sur la figure 1 et destinée à être orientée vers le tronc de l'utilisateur et une face externe 11 visible sur la figure 1 et opposée à la face interne.

La ceinture 10 comprend un premier panneau latéral 12 et un deuxième panneau latéral 14.

Les premier et deuxième panneaux latéraux 12, 14 sont destinés à entourer ensemble au moins une partie du corps de l'utilisateur, plus particulièrement le tronc.

Les premier et deuxième panneaux latéraux 12, 14 sont asymétriques l'un par rapport à l'autre par rapport à la ligne centrale D.

Plus particulièrement, l'un des panneaux latéraux 14 présente une dimension selon une direction perpendiculaire à la direction principale X, dite direction d'extension Y, strictement supérieure à celle de l'autre des panneaux latéraux 12.

Le rapport de la dimension de l'un des panneaux latéraux 14 selon la direction d'extension Y sur la dimension de l'autre des panneaux latéraux 12 selon la direction d'extension Y est, par exemple, supérieure ou égale à 1,1.

Cela permet notamment d'avoir une plage de réglage importante, en particulier pour adapter la ceinture en fonction des mensurations d'un utilisateur, et/ou une plage de serrage importante, en fonction de l'effort nécessaire pour soulager l'utilisateur.

Chaque panneau latéral 12, 14 présente une extrémité dorsale 16, 18 et une extrémité distale 20, 22 à l'opposé de l'extrémité dorsale 16, 18. L'extrémité dorsale 16, 18 et l'extrémité distale 20, 22 sont opposées selon la direction d'extension Y.

Les extrémités dorsales 16, 18 sont agencées de part et d'autre de la ligne centrale D, et ainsi prévues pour être placées de part et d'autre de la colonne vertébrale, plus particulièrement à proximité de la colonne, d'un utilisateur lorsque la ceinture est portée. Les extrémités dorsales 16, 18 sont prévues pour être placées symétriquement l'une par rapport à l'autre par rapport à ladite colonne vertébrale.

Les extrémités dorsales 16, 18 des premier et deuxième panneaux latéraux 12, 14, et ainsi les panneaux latéraux 12, 14, sont en outre reliées l'une à l'autre, par exemple, via un mécanisme de serrage 40 qui sera décrit plus en détail par la suite.

Alternativement, les extrémités dorsales 16, 18 sont reliées par un autre système d'attache ou sont solidaires de sorte que le premier panneau latéral et le deuxième panneau latéral forment une seule pièce.

Les extrémités distales 20, 22 des premier et deuxième panneaux latéraux 12, 14 sont adaptées pour être fixées l'une à l'autre de manière réversible.

Les panneaux latéraux 12, 14 étant asymétriques par rapport à la ligne centrale D, les extrémités distales 20, 22 sont configurées pour être placées asymétriquement autour de l'utilisateur, plus particulièrement sur la partie ventrale de l'utilisateur.

Chaque panneau latéral 12, 14 comprend, plus particulièrement, une portion dorsale 24, 26 prévue pour s'étendre à côté de la colonne vertébrale d'un utilisateur lorsque la ceinture 10 est portée, une portion distale 28, 30 et une portion intermédiaire 32, 34 entre la portion dorsale 24, 26 et la portion distale 28, 30.

La portion dorsale 24, 26, la portion intermédiaire 32, 34 et la portion dorsale 24, 26 d'un panneau latéral 12, 14 sont alignés selon la direction d'extension Y.

Chaque portion dorsale 24, 26 définit l'extrémité dorsale 16, 18 correspondante et chaque portion distale 28, 30 l'extrémité distale 20, 22 correspondante.

Les portions dorsales 24, 26 sont sensiblement symétriques l'une par rapport à l'autre par rapport à la ligne centrale D.

Les portions dorsales 24, 26 ne sont pas élastiques, c'est-à-dire qu'elles ne peuvent pas être étirées.

Chaque portion dorsale 24, 26 comprend au moins un élément de maintien 36, 38.

Les éléments de maintien 36, 38 des portions dorsales 24, 26 des panneaux latéraux sont placés symétriquement de part et d'autre de la ligne centrale D.

Dans le mode de réalisation représenté, chaque élément de maintien 36, 38 est ici une baleine s'étendant sensiblement parallèlement à l'extrémité dorsale 16, 18 correspondante.

Alternativement, chaque portion dorsale 24, 26 comprend deux éléments de maintien, ici des baleines, les éléments de maintien des portions dorsales s'étendant symétriquement de part et d'autre de la ligne centrale D. Chaque portion dorsale 24, 26 comprend, par exemple, un premier élément de maintien à proximité et s'étendant sensiblement parallèlement à l'extrémité dorsale 16, 18 correspondante et un deuxième élément de maintien plus éloigné de l'extrémité dorsale 16, 18 que ne l'est le premier élément de maintien. Le deuxième élément de maintien n'est pas ici parallèle à l'extrémité dorsale.

Les portions intermédiaires 32, 34 sont sensiblement symétriques l'une par rapport à l'autre par rapport à la ligne centrale D.

Les portions intermédiaires 32, 34 sont élastiques. Elles sont, par exemple, tissées ou tricotées à partir de fils élastiques.

On entend ici par « élastique » que la ceinture 10 dans lesdites portions est apte à être étirée d'au moins 10% sans endommagement et à retrouver ses dimensions initiales en l'absence d'effort d'étirement.

Les portions distales 28, 30 sont asymétriques l'une par rapport à l'autre par rapport à la ligne centrale D. Plus particulièrement, l'une des portions distales 30 présente une dimension selon la direction d'extension Y strictement supérieure à celle de l'autre des portions distales 28.

Le rapport entre la dimension selon la direction d'extension Y de l'une des portions distales 30 et celle de l'autre des portions distales 28 est ici strictement supérieur à 1,25, plus particulièrement supérieur ou égal à 1,50. Ledit rapport dépend, par exemple, de la plage de réglage et/ou des dimensions de la fixation réversible des extrémités distales décrite ci-après.

Les portions distales 28, 30 ici ne sont pas élastiques, c'est-à-dire qu'elles ne peuvent pas être étirées.

Pour permettre la fixation réversible des extrémités distales entre elles, les portions distales 28, 30 sont, par exemple, chacune pourvues d'au moins une bande autoagrippante complémentaire du type boucles et crochets, à l'une extrémité sur la face interne et à l'autre extrémité sur la face externe.

Plus particulièrement, la face externe de la portion distale 28, 30 d'au moins un des panneaux latéraux 12, 14, plus particulièrement des premier et deuxième panneaux latéraux, est formée de l'un des types boucles ou crochet.

Un des panneaux latéraux est pourvu sur sa face interne d'une partie de l'autre des types boucles ou crochet.

Les bandes autoagrippantes coopèrent entre elles lorsque la ceinture est fermée autour du tronc de l'utilisateur.

Le premier panneau latéral 12 et le second panneau latéral 14, plus particulièrement les portions distales 28, 30, sont, en outre, chacun pourvus d'un élément de préhension 42, 44 pour l'utilisateur pour positionner la ceinture autour de l'utilisateur.

Les éléments de préhension 42, 44 sont disposés symétriquement sur la ceinture 10 par rapport à la ligne centrale D.

Le rapport entre la distance moyenne entre la ligne centrale D et chaque extrémité distale 20, 22 et la distance entre la ligne centrale D et chaque élément de préhension 42, 44 est ici compris entre 1,5 et 1,75.

Le rapport entre la distance moyenne entre la ligne centrale D et l'extrémité distale 20 du premier panneau latéral 12 et la distance entre la ligne centrale D et l'élément de préhension 42 correspondant est ici supérieur ou égal à 1.

Le rapport entre la distance moyenne entre la ligne centrale D et l'extrémité distale 22 du deuxième panneau latéral 14 et la distance entre la ligne centrale D et l'élément de préhension 44 correspondant est ici inférieur ou égal à 2.

Chaque élément de préhension 42, 44 comprend une bande textile s'étendant entre une première extrémité 46 et une deuxième extrémité 48.

La bande textile est configurée pour qu'un utilisateur soit apte à glisser une main ou un doigt, tel qu'un pouce, entre la bande textile et la face externe du panneau latéral correspondant.

La bande textile est liée au premier ou deuxième panneau latéral respectif 12, 14 aux première et deuxième extrémités 46, 48.

La bande textile est ici cousue à la portion distale 28, 30 du panneau latéral correspondant 12, 14.

La bande textile présente une face externe, avantageusement formée du type boucles ou crochets identique à celui de la face externe 11 du panneau latéral correspondant 12, 14.

La bande textile est ici un quadrilatère dont deux côtés opposés forment les première et deuxième extrémités 46, 48.

La bande textile est libre du premier ou deuxième panneau latéral respectif 12, 14 en dehors des première et deuxième extrémités 46, 48 de la bande textile.

Les première et deuxième extrémités 46, 48 de la bande textile sont sensiblement alignées selon la direction principale.

Les première et deuxième extrémités 46, 48 de la bande textile s'étendent selon des axes respectifs non parallèles l'un à l'autre.

La dimension de la bande textile selon la direction principale diminue en s'approchant de l'extrémité distale 20, 22 du premier ou deuxième panneau latéral respectif 12, 14.

La dimension de la bande textile selon la direction principale à son extrémité la plus proche de l'extrémité dorsale 16, 18 est, par exemple, comprise entre 3 cm et 15 cm pour le passage d'un doigt ou d'une main, plus particulièrement comprise entre 8 cm et 15 cm, notamment pour le passage d'une main.

La dimension de la bande textile selon la direction principale à son extrémité la plus proche de l'extrémité distale 20, 22 est, par exemple, comprise entre 2 cm et 10 cm pour le passage d'un doigt ou d'une main, plus particulièrement comprise entre 5 cm et 10 cm, notamment pour le passage d'une main.

Cela permet notamment d'avoir des dimensions particulièrement adaptées pour qu'une main d'un utilisateur puisse se glisser.

Le mécanisme de serrage 40 mentionné précédemment est maintenant décrit plus en détail.

Le mécanisme de serrage 40 comprend des guides de câble 50, 52, 54, 56, un câble 58, plus particulièrement un unique câble, un premier élément de poignée 60 et un deuxième élément de poignée 62.

Plus particulièrement, le mécanisme de serrage 40 comprend, sur chacune des portions dorsales 24, 26 des premier et deuxième panneaux latéraux 12, 14, une rangée supérieure de guides de câble 50, 52 et une rangée inférieure de guides de câble 54, 56.

La rangée supérieure de guides de câble 50 du premier panneau latéral 12 est espacée de la rangée inférieure de guides de câble 54 du premier panneau latéral 12 par un espace. L'espace est supérieur à 2 millimètres (mm). Elles sont sensiblement alignées selon la direction principale.

L'espace est avantageusement supérieur à la distance entre deux guides adjacents de chacune des rangées de guides 50,54, ici plus particulièrement supérieur à 35 millimètres.

La rangée supérieure de guides de câble 52 du deuxième panneau latéral 14 est espacée de la rangée inférieure de guides de câble 56 du deuxième panneau latéral 14 par un espace. L'espace est supérieur à 2 millimètres (mm). Elles sont sensiblement alignées selon la direction principale.

L'espace est avantageusement supérieur à la distance entre deux guides adjacents de chacune des rangées de guides 52,56, ici plus particulièrement supérieur à 35 millimètres.

Chaque portion dorsale 24, 26 est, par exemple, pourvue d'une plaque supérieure et d'une plaque inférieure, la rangée supérieure de guides de câble 50, 52 étant agencée sur la plaque supérieure et la rangée inférieure de guides de câble 54, 56 étant agencée sur la plaque inférieure.

Les plaques d'un même panneau latéral 12, 14 sont espacées d'une distance comprise entre 1 millimètre (mm) et 20 centimètres (cm).

Chaque guide de câble est, par exemple, réalisé d'une pièce avec la plaque correspondante, ce qui permet de réduire le nombre d'éléments constitutifs du mécanisme de serrage. Selon un autre mode de réalisation, les guides de câble sont des pièces rapportées, par exemple, fixées sur la plaque.

Chaque guide de câble délimite ici un passage pour le guidage du câble.

Chaque guide de câble 50, 52, 54, 56 comprend, par exemple, un ergot fixe autour duquel le câble est prévu pour être enfilé.

L'ergot fixe s'étend en saillie de la plaque correspondante.

L'ergot est cylindrique et s'étend selon un axe sensiblement perpendiculaire au plan dans lequel s'étend la plaque de sorte qu'un câble, arrivant sur l'ergot selon une direction initiale, passe autour de l'ergot et sort de l'ergot selon une direction formant un angle non nul avec la direction initiale.

Chaque guide de câble comprend en outre un moyen de retenue du câble autour de l'ergot. Le moyen de retenue est, par exemple, une rainure, un tube ou une butée supérieure et/ou radiale.

Alternativement, chaque guide de câble est formé d'une poulie.

Le câble 58 comprend une première extrémité 68 et une deuxième extrémité 70.

Le câble 58 est enfilé à travers les guides de câble des rangées supérieure et inférieure du premier panneau latéral 12 et du deuxième panneau latéral 14. Plus particulièrement, le câble 58 est enfilé alternativement à travers les guides de câble du premier panneau latéral 12 et du deuxième panneau latéral 14, plus particulièrement à travers les rangées de guides supérieures 50, 52 d'une part et les rangées de guides inférieures 54, 56 d'autre part.

Cela forme un laçage croisé du câble 58 entre le premier panneau latéral 12 et le deuxième panneau latéral 14, plus particulièrement d'une part dans une partie inférieure et d'autre part dans une partie supérieure de la ceinture.

Le câble 58 est relié aux premier et deuxième éléments de poignée 60, 62, de sorte que les extrémités dorsales 16, 18 des premier et deuxième panneaux latéraux 12, 14 sont rapprochées par serrage du câble 58 autour des guides de câble des rangées supérieure et inférieure 50, 52, 54, 56 lorsqu'un des éléments de poignée 60, 62 est tiré.

Chaque élément de poignée 60, 62 comprend, par exemple, au moins une ouverture 64, 66 pour permettre la préhension dudit élément de poignée 60, 62 pour permettre à un utilisateur de tirer sur l'élément de poignée 60, 62.

Les première et deuxième extrémités du câble 68, 70 sont fixées de manière fixe au premier élément de poignée 60, le câble 58 étant enfilé de manière coulissante à travers le deuxième élément de poignée 62.

Plus particulièrement, chaque élément de poignée 60, 62 présente deux orifices traversants 72.

Le câble 58 présente un élargissement au niveau de la première extrémité 68 et de la deuxième extrémité 70 prévu pour maintenir chaque extrémité 68, 70 fixe au niveau d'un orifice traversant respectif 72.

Cela est, par exemple, obtenu par nouage dudit câble aux extrémités 68, 70 après passage de chaque extrémité à travers un orifice traversant respectif du premier élément de poignée 60.

Alternativement, les extrémités 68, 70 du câble 58 sont fixées ensemble après passage de chaque extrémité du câble par un orifice traversant respectif 72. Les extrémités du câble sont alors susceptibles de coulisser par rapport au premier élément de poignée 60 d'une distance inférieure à la distance entre les deux orifices traversants. Il est considéré ici que les extrémités du câble 68, 70 sont alors sensiblement fixes par rapport au premier élément de poignée 60.

Le câble 58 traverse, par ailleurs, successivement les deux orifices traversants du deuxième élément de poignée 62. Cela permet notamment au câble de pouvoir coulisser à travers les deux orifices traversants.

Un tel coulissement du câble par rapport au deuxième élément de poignée 62 permet notamment d'ajuster le rapport entre la longueur de câble 58 dans une partie supérieure de la ceinture 10 et la longueur de câble 58 dans une partie inférieure de la ceinture 10, de manière à mieux ajuster la ceinture 10 à la morphologie de l'utilisateur.

En effet, en augmentant la longueur de câble 58 dans une partie supérieure, cela écarte d'autant les extrémités dorsales 16, 18 dans la partie supérieure de la ceinture par rapport à la partie inférieure, de sorte que cela est particulièrement adapté pour une morphologie en V. Inversement, en diminuant la longueur de câble 58 dans une partie supérieure, cela rapproche d'autant les extrémités dorsales 16, 18 dans la partie supérieure de la ceinture 10 par rapport à la partie inférieure, de sorte que cela est particulièrement adapté pour une morphologie en ∧.

Une méthode pour mettre en place une ceinture orthopédique selon l'invention va maintenant être décrite.

Un utilisateur saisit la ceinture 10 en utilisant les éléments de préhension 42, 44, notamment en passant chacune de ses mains dans l'un des éléments de préhension, plus particulièrement entre la ceinture et la bande textile de l'élément de préhension.

L'utilisateur place la ceinture 10 autour de son tronc en plaçant la ligne centrale D contre la colonne vertébrale. Cela est aisé en plaçant les éléments de préhension de part et d'autre de son tronc de manière symétrique entre son côté droit et son côté gauche.

Une fois la ceinture correctement placée, l'utilisateur attache la ceinture 10 en fixant les extrémités distales 20, 22 l'une à l'autre.

La présence des éléments de préhension permet donc de symétriser la prise pour l'utilisateur qui possède alors un repère pour placer correctement la ligne centrale de la ceinture contre sa colonne vertébrale, sans avoir besoin d'aide extérieur.

Le fait de placer correctement la ceinture permet notamment que la ceinture assure l'effet souhaité sur l'utilisateur.

## Revendications

1. Ceinture orthopédique (10) pour un utilisateur, comprenant un premier panneau latéral (12) et un deuxième panneau latéral (14), chacun du premier panneau latéral (12) et du deuxième panneau latéral (14) présentant une extrémité dorsale (16, 18) et une extrémité distale (20, 22), les premier et deuxième panneaux latéraux (12, 14) étant destinés à entourer ensemble au moins une partie du corps de l'utilisateur, la ceinture (10) définissant une ligne centrale (D) adaptée pour être placée contre la colonne vertébrale de l'utilisateur, la ligne centrale (D) définissant une direction principale,
les extrémités dorsales (16, 18) des premier et deuxième panneaux latéraux (12, 14) étant reliées l'une à l'autre,
les extrémités distales (20, 22) des premier et deuxième panneaux latéraux (12, 14) étant adaptées pour être fixées l'une à l'autre de manière réversible,
le premier panneau latéral (12) et le second panneau latéral (14) étant chacun pourvus d'un élément de préhension (42, 44) pour l'utilisateur pour positionner la ceinture (10) autour de l'utilisateur,
les éléments de préhension (42, 44) étant disposés symétriquement sur la ceinture (10) par rapport à la ligne centrale (D), **caractérisée en ce que** les premier et deuxième panneaux latéraux (12, 14) sont asymétriques l'un par rapport à l'autre par rapport à la ligne centrale (D), de sorte que les extrémités distales (20, 22) sont configurées pour être placées asymétriquement autour de l'utilisateur.

2. Ceinture orthopédique selon la revendication 1, dans laquelle le rapport entre la distance moyenne entre la ligne centrale (D) et chaque extrémité distale (20, 22) et la distance entre la ligne centrale (D) et chaque élément de préhension (42, 44) est compris entre 1,5 et 1,75.

3. Ceinture orthopédique selon la revendication 1 ou 2, dans laquelle chaque élément de préhension (42, 44) comprend une bande textile s'étendant entre une première extrémité (46) et une deuxième extrémité (48), la bande textile étant liée au premier ou deuxième panneau latéral respectif (12, 14) aux première et deuxième extrémités (46, 48).

4. Ceinture orthopédique selon la revendication 3, dans laquelle la bande textile est libre du premier ou deuxième panneau latéral respectif (12, 14) en dehors des première et deuxième extrémités (46, 48) de la bande textile.

5. Ceinture orthopédique selon la revendication 3 ou 4, dans laquelle les première et deuxième extrémités (46, 48) de la bande textile sont sensiblement alignées selon la direction principale.

6. Ceinture orthopédique selon la revendication 5, dans laquelle les première et deuxième extrémités (46, 48) de la bande textile s'étendent selon des axes respectifs non parallèles l'un à l'autre, la dimension de la bande textile selon la direction principale diminuant en s'approchant de l'extrémité distale (20, 22) du premier ou deuxième panneau latéral respectif (12, 14).

7. Ceinture orthopédique selon l'une quelconque des revendications 1 à 6, comprenant un mécanisme de serrage (40) comprenant :
- une rangée supérieure de guides de câble (50, 52) sur chacune des extrémités dorsales (16, 18) des premier et deuxième panneaux latéraux (12, 14),
- une rangée inférieure de guides de câble (54, 56) sur chacune des extrémités dorsales (16, 18) des premier et deuxième panneaux latéraux (12, 14),
- un câble (58) enfilé à travers les guides de câble des rangées supérieure et inférieure (50, 52, 54, 56) du premier panneau latéral (12) et du deuxième panneau latéral (14), le câble (58) étant enfilé alternativement à travers les guides de câble du premier panneau latéral (12) et du deuxième panneau latéral (14),
- un premier et un deuxième éléments de poignée (42, 44),
le câble (58) étant relié aux premier et deuxième éléments de poignée (42, 44), de sorte que les extrémités dorsales (16, 18) des premier et deuxième panneaux latéraux (12, 14) sont rapprochées par serrage du câble (58) autour des guides de câble des rangées supérieure et inférieure (50, 52, 54, 56) lorsqu'au moins un du premier et du deuxième éléments de poignée (42, 44) est tiré,
les extrémités (68, 70) du câble (58) étant fixées de manière au moins sensiblement fixe au premier élément de poignée (42), le câble (58) étant enfilé de manière coulissante à travers le deuxième élément de poignée (44).

8. Ceinture orthopédique selon la revendication 7, dans laquelle la rangée supérieure de guides de câble (50) du premier panneau latéral (12) est espacée de la rangée inférieure de guides de câble (54) du premier panneau latéral (12) par un espace et la rangée supérieure de guides de câble (52) du deuxième panneau latéral (14) est espacée de la rangée inférieure de guides de câble (56) du deuxième panneau latéral (14) par un espace.

9. Ceinture orthopédique selon la revendication 7 ou 8, dans laquelle le câble (58) est unique.

10. Méthode de mise en place d'une ceinture orthopédique selon l'une quelconque des revendications 1 à 9, comprenant les étapes suivantes :
- saisie par un utilisateur de la ceinture (10) par les éléments de préhension (42, 44),
- placement de la ceinture (10) autour d'une partie du corps de l'utilisateur par placement des éléments de préhension (42, 44) symétriquement de part et d'autre de ladite partie du corps, et
- attache de la ceinture (10) autour du corps de l'utilisateur par fixation des extrémités distales (20, 22) l'une à l'autre.

## Patentansprüche

1. Orthopädisches Band (10) für einen Benutzer, umfassend ein erstes Seitenfeld (12) und ein zweites Seitenfeld (14), wobei das erste Seitenfeld (12) und das zweite Seitenfeld (14) ein dorsales Ende (16, 18) und ein distales Ende (20, 22) aufweist, wobei das erste und das zweite Seitenfeld (12, 14) dazu bestimmt sind, zusammen mindestens einen Teil des Körpers des Benutzers zu umgeben, wobei das Band (10) eine Mittellinie (D) definiert, die dazu geeignet ist, gegen die Wirbelsäule des Benutzers platziert zu werden, wobei die Mittellinie (D) eine Hauptrichtung definiert, die dorsalen Enden (16, 18) des ersten und zweiten Seitenfelds (12, 14) miteinander verbunden sind,
die distalen Enden (20, 22) des ersten und zweiten Seitenfelds (12, 14) so angepasst sind, dass sie reversibel aneinander befestigt werden können, wobei das erste Seitenfeld (12) und das zweite Seitenfeld (14) jeweils mit einem Greifelement (42, 44) für den Benutzer versehen sind, um das Band (10) um den Benutzer herum zu positionieren,
die Greifelemente (42, 44) symmetrisch auf dem Band (10) in Bezug auf die zentrale Liane (D) angeordnet sind, **dadurch gekennzeichnet, dass** das erste und zweite Seitenfeld (12, 14) asymmetrisch zueinander angeordnet sind, in Bezug auf die Mittellinie (D) zueinander asymmetrisch sind, so dass die distalen Enden (20, 22) so konfiguriert sind, dass sie asymmetrisch um den Benutzer herum angeordnet werden können.

2. Orthopädisches Band nach Anspruch 1, wobei das Verhältnis zwischen dem durchschnittlichen Abstand zwischen der Mittellinie (D) und jedem distalen Ende (20, 22) und dem Abstand zwischen der Mittellinie (D) und jedem Greifelement (42, 44) zwischen 1,5 und 1,75 liegt.

3. Orthopädisches Band nach Anspruch 1 oder 2, wobei jedes Greifelement (42, 44) ein Textilband umfasst, das sich zwischen einem ersten Ende (46) und einem zweiten Ende (48) erstreckt, wobei das Textilband an dem ersten und zweiten Ende (46, 48) mit dem jeweiligen ersten oder zweiten Seitenfeld (12, 14) verbunden ist.

4. Orthopädisches Band nach Anspruch 3, wobei das Textilband außerhalb des ersten und zweiten Endes (46, 48) des Textilbandes frei von dem jeweiligen ersten oder zweiten Seitenfeld (12, 14) ist.

5. Orthopädisches Band nach Anspruch 3 oder 4, bei dem das erste und das zweite Ende (46, 48) des Textilbands im Wesentlichen in der Hauptrichtung ausgerichtet sind.

6. Orthopädisches Band nach Anspruch 5, bei dem sich das erste und das zweite Ende (46, 48) des Textilbands entlang jeweiliger, nicht parallel zueinander verlaufender Achsen erstrecken, wobei die Abmessung des Textilbands in der Hauptrichtung abnimmt, wenn man sich dem distalen Ende (20, 22) des jeweiligen ersten oder zweiten Seitenfelds (12, 14) nähert.

7. Orthopädisches Band nach einem der Ansprüche 1 bis 6, das einen Festziehmechanismus (40) enthält, der Folgendes umfasst:
- eine obere Reihe von Kabelführungen (50, 52) an jedem der dorsalen Enden (16, 18) des ersten und zweiten Seitenfelds (12, 14),
- eine untere Reihe von Kabelführungen (54, 56) an jedem der dorsalen Enden (16, 18) des ersten und zweiten Seitenfelds (12, 14),
- ein Kabel (58), das durch die Kabelführungen der oberen und unteren Reihen (50, 52, 54, 56) des ersten Seitenfelds (12) und des zweiten Seitenfelds (14) gefädelt ist, wobei das Kabel (58) abwechselnd durch die Kabelführungen des ersten Seitenfelds (12) und des zweiten Seitenfelds (14) gefädelt wird,
- ein erstes und ein zweites Griffelement (42, 44), wobei das Kabel (58) mit dem ersten und dem zweiten Griffelement (42, 44) verbunden ist, so dass die dorsalen Enden (16, 18) des ersten und des zweiten Seitenfelds (12, 14) zusammengebracht werden, indem das Kabel (58) um die Kabelführungen der oberen und unteren Reihe (50, 52, 54, 56) festgezogen wird, wenn mindestens eines der ersten und zweiten Griffelemente (42, 44) gezogen wird,
die Enden (68, 70) des Kabels (58) zumindest im Wesentlichen fest an dem ersten Griffelement (42) befestigt sind, wobei das Kabel (58) verschiebbar durch das zweite Griffelement (44) gefädelt ist.

8. Orthopädisches Band nach Anspruch 7, wobei die obere Reihe von Kabelführungen (50) des ersten Seitenfelds (12) von der unteren Reihe von Kabelführungen (54) des ersten Seitenfelds (12) durch einen Zwischenraum beabstandet ist und die obere Reihe von Kabelführungen (52) des zweiten Seitenfelds (14) von der unteren Reihe von Kabelführungen (56) des zweiten Seitenfelds (14) durch einen Zwischenraum beabstandet ist.

9. Orthopädisches Band nach Anspruch 7 oder 8, wobei das Kabel (58) einzig ist.

10. Verfahren zur Anbringung eines orthopädischen Bandes nach einem der Ansprüche 1 bis 9, das die folgenden Schritte umfasst:
- Greifen des Bands (10) durch einen Benutzer an den Greifelementen (42, 44),
- Platzieren des Bands (10) um einen Teil des Körpers des Benutzers durch Platzieren der Greifelemente (42, 44) symmetrisch auf beiden Seiten des Körperteils, und
- Befestigen des Bands (10) um den Körper des Benutzers, indem die distalen Enden (20, 22) aneinander befestigt werden.

## Claims

1. An orthopedic belt (10) for a user, comprising a first side panel (12) and a second side panel (14), each of the first side panel (12) and the second side panel (14) having a dorsal end (16, 18) and a distal end (20, 22), the first and second side panels (12, 14) being designed to jointly surround at least one portion of the user's body, the belt (10) defining a central line (D) adapted to be placed against the user's spine, the central line (D) defining a primary direction,
the dorsal ends (16, 18) of the first and second side panels (12, 14) being connected to each other,
the distal ends (20, 22) of the first and second side panels (12, 14) being adapted to be reversibly attached to each other,
the first side panel (12) and the second side panel (14) each being provided with a user gripping element (42, 44) for positioning the belt (10) around the user,
the gripping elements (42, 44) being arranged symmetrically on the belt (10) with respect to the central line (D), **characterized in that** the first and second side panels (12, 14) are asymmetrical in relation to one another with respect to the central line (D), such that the distal ends (20, 22) are configured to be placed asymmetrically around the user.

2. The orthopedic belt according to claim 1, wherein the ratio of the average distance between the central line (D) and each distal end (20, 22) to the distance between the central line (D) and each gripping element (42, 44) is between 1.5 and 1.75.

3. The orthopedic belt according to claim 1 or 2, wherein each gripping element (42, 44) comprises a textile strip extending between a first end (46) and a second end (48), the textile strip being linked to the respective first or second side panel (12, 14) at the first and second ends (46, 48).

4. The orthopedic belt according to claim 3, wherein the textile strip is free of the respective first or second side panel (12, 14) outside of the first and second ends (46, 48) of the textile strip.

5. The orthopedic belt according to claim 3 or 4, wherein the first and second ends (46, 48) of the textile strip are substantially aligned along the primary direction.

6. The orthopedic belt according to claim 5, wherein the first and second ends (46, 48) of the textile strip extend along respective axes not parallel to each other, the dimension of the textile strip along the principal direction decreasing as it approaches the distal end (20, 22) of the respective first or second side panel (12, 14).

7. The orthopedic belt according to any one of claims 1 to 6, comprising a tightening mechanism (40) comprising:
- an upper row of cable guides (50, 52) on each of the dorsal ends (16, 18) of the first and second side panels (12, 14),
- a lower row of cable guides (54, 56) on each of the dorsal ends (16, 18) of the first and second side panels (12, 14)
- a cable (58) threaded through the upper and lower rows of cable guides (50, 52, 54, 56) of the first side panel (12) and the second side panel (14), the cable (58) being threaded alternately through the cable guides of the first side panel (12) and the second side panel (14)
- a first and a second handle elements (42, 44),
the cable (58) being connected to the first and second handle elements (42, 44) such that the dorsal ends (16, 18) of the first and second side panels (12, 14) are brought together by tightening the cable (58) around the upper and lower row cable guides (50, 52, 54, 56) when at least one of the first and second handle elements (42, 44) is pulled,
the ends (68, 70) of the cable (58) being at least substantially fixedly attached to the first handle element (42), the cable (58) being slidably threaded through the second handle element (44).

8. The orthopedic belt according to claim 7, wherein the upper row of cable guides (50) of the first side panel (12) is spaced from the lower row of cable guides (54) of the first side panel (12) by a space and the upper row of cable guides (52) of the second side panel (14) is spaced from the lower row of cable guides (56) of the second side panel (14) by a space.

9. The orthopedic belt according to claim 7 or 8, wherein the cable (58) is unique.

10. A method of placing an orthopedic belt according to any one of claims 1 to 9, comprising the following steps:
- a user grasping the belt (10) by the gripping elements (42, 44),
- placing the belt (10) around a portion of the user's body by placing the gripping elements (42, 44) symmetrically on either side of said portion the body, and
- securing the belt (10) around the user's body by fastening the distal ends (20, 22) to each other.
